# EUROPEAN PATENT APPLICATION

(11) **EP 4 155 390 A1**
(43) Date of publication of application: **29.03.2023**
(21) Application number: 20936500.6
(22) Date of filing: 19.05.2020
(51) Int. Cl.: C12N 5/079, C12N 5/0793, C12Q 1/02

(54) **SERUM-FREE INDUCTION METHOD FOR SENSORY NEURON CELL**

(71) Applicant: Iregene Therapeutics Ltd, Wuhan, Hubei 430074 (CN)
(72) Inventor: WEI, Jun, Wuhan, Hubei 430074 (CN); CAI, Meng, Wuhan, Hubei 430074 (CN)
(74) Representative: Nederlandsch Octrooibureau
(86) International application number: PCT/CN2020/091041
(87) International publication number: WO 2021/232234

(57) **Abstract**

Provided is a human-derived sensory neuron induction culture system. A combination of a small molecule inhibitor LY2157299 and a growth factor is added into a serum-free basal medium. Compared with an induction method involving serum, not only is the efficiency of inducing pluripotent stem cells into sensory neurons greatly improved, but the expression of a variety of ion channel proteins is also significantly improved, thereby achieving successful induction of multiple induced pluripotent stem cells from different sources into sensory neurons.

## Description

### FIELD OF THE INVENTION

The present invention belongs to biological field. Specifically, it relates to a method for inducing pluripotent stem cells into sensory neuron cells, an induction medium and uses thereof.

### BACKGROUND OF THE INVENTION

In 2006, Shinya Yamanaka's team invented a "cocktail" composed of four transcription factors (i.e., OCT4, SOX2, KLF4 and c-Myc), which could successfully reprogram terminally differentiated skin fibroblasts into stem cells with differentiation pluripotency. Such stem cells are called induced pluripotent stem cells (Cell, 2006, 124(4) pp.663-676; Cell, 2007, 131(5) pp.861-872). These stem cells have a similar differentiation potential to embryonic stem cells, and can form the three most basic germ layers in human development: ectoderm, mesoderm and endoderm, and can eventually form a variety of somatic cells. This invention breaks through the ethical limitation of using human embryonic stem cells in medicine, can solve the problem of immune rejection in cell transplantation therapy, and greatly expands the application potential of stem cell technology in clinical medicine.

Peripheral sensory neurons, known as nociceptors, can sense extreme temperatures and pressures, as well as detect damage-related chemicals; and can further convert these stimuli into electrical signals, which are transmitted to the central nervous system over long distances. The diversity of peripheral sensory neuron activation and information transmitted therefrom also contributes to the diversity of pain levels and types. Therefore, peripheral sensory neurons are the focus of pain mechanism research and development of analgesic medicines. Taking the analgesic medicines in nervous system medicines as an example, pain is essential to protect organs from serious injury or damage. Depending on the type, location, and intensity of pain generation, this information is transmitted to the spinal cord via sensory neurons in the DRG or trigeminal ganglia, and ultimately to the thalamus and cerebral cortex. The circuit mechanisms, biochemical responses and molecular interactions of pain are complex and highly variable. Alterations in nociceptor pathway can lead to allergic and chronic pain, which affects 20% of adults, including arthritis, neuralgia caused by infection, and cancer pain. Therefore, understanding the signaling and modulation of pain is important to develop better analgesic medicines. Non-human mammalian neurons (usually derived from rats) are generally used in traditional methods for medicine screening. However, these animal cell culture models are quite different from human physiology and cannot fully represent human physiological, pharmacological and pharmacodynamic responses. Therefore, many medicines in human clinical trials do not have similar effects to animal experiments, thereby leading to unnecessary risks and waste of early research and development.

Based on the great differentiation potential of induced pluripotent cells, human pluripotent stem cells are also widely used in medicine screening. By using sensory neurons differentiated from human pluripotent stem cells, it is possible to gain an in-depth understanding of the working principle of sensory neurons and how neurons interact with medicines to generate therapeutic mechanisms, thereby further discovering new therapeutic targets.

In the field of sensory nervous system, primary cell lines, immortalized cell lines, and trans-ion channel gene cell lines have been used for *in vitro* studies, but there are considerable limitations for the use of primary cells from patient/ body donations, for example, the limited number of *in vitro* proliferation limits the use of primary cells in high-throughput screening; while immortalized cell lines and transgenic cell lines are quite different from primary cells in many indices, and cannot accurately represent the physiological function of natural cells. Therefore, human induced pluripotent stem cell (iPSC)-derived sensory neurons serve as a novel alternative that can be broadly applied to test new medicine candidates. From pluripotent stem cells to neural stem cells, there are currently two most widely used induction methods.The first is a two-step method: firstly, cells aggregate into clusters to form embryonic bodies, and then cells are further induced to become neurons. The disadvantage of this method is that the cell differentiation is not synchronized, and the purity of neuronal cells is not high; in addition, the use of animal-derived growth factors also restricts the clinical use of neuronal cells. The second method is Dual SMAD inhibition (Nat Biotechnol, 2009, 05; 27(5): 485). The neural stem cells obtained by this method can differentiate into other types of neuron cells. The mechanism of the method is to simulate the signaling pathway in early embryonic development by inhibiting the BMP and TGFB pathways, thereby inducing the generation of neural stem cells. In this way, sensory neurons can be induced in the presence of Knockout Serum Replacement (Nat Biotechnol., 2013, 30(7): 715-720). Feeder cells are not used in this method, but Knockout Serum Replacement is still needed, which may have a certain interference effect on medicine screening. In addition, some non-target cells are mixed in the induced cells, which may affect the purity of cells.

The present application discloses a serum-free and feeder cell-free directional induction method, which combines pure chemical factor induction and growth factor, simulates the signal pathway of early embryonic development, and can induce directional differentiation of human induced pluripotent stem cells from various sources into sensory neurons which express a variety of ion channel proteins, and have electrophysiological functions, thereby greatly expanding the use of induced nerve cells in medicine screening.

### SUMMARY OF THE INVENTION

The present disclosure relates to molecules and culture medium for inducing into sensory neuron cells, and correspondingly induced and cultured sensory nerve precursor cells and sensory neuron cells.

Specifically, the present disclosure relates to the following aspects:
1. Use of LY2157299, RepSox, SB5253334 or TEW7179 in the induction of pluripotent stem cells to differentiate into sensory nerve precursor cells and sensory neuron cells.
2. The use of item 1, wherein the dosage of LY2157299, RepSox, SB5253334 or TEW7179 is 55nM-25µM, preferably 100nM, 300nM, 500nM, 700nM, 900nM, 1µM, 2.5µM, 5µM, 7.5µM, 10µM, 12.5µM, 15µM, 17.5µM, 20µM or 22µM.
3. Use of human-NT3 or CHIR98014 in culturing sensory neurons.
4. The use of item 3, wherein the dosage of human-NT3 is 0.1-49ng/ml, or the amount of CHIR98014 is 0.5nM-3µM.
5. An induction medium, characterized in that a basal medium is formed by 50% (v/v) DMEM medium and 50% (v/v) neural basal medium, then the basal medium is supplemented with 0.5%-2% (v/v) N2 supplement (preferably 0.7%, 0.9%, 1.0%, 1.1%, 1.2%, 1.3%, 1.4%, 1.5%, 1.6%, 1.7% (v/v)), 0.2%-3.1% (v/v) GS21 supplement or B27 supplement (preferably 0.4%, 0.6%, 0.8%, 1.0%, 1.2%, 1.4%, 1.6%, 1.8%, 2.0%, 2.2%, 2.4%, 2.6%, 2.8%, 3.0% (v/v)), 0.1-15 mM HEPES buffer (preferably 1, 5, 10 mM), 5-22 mM glycine-glutamine monohydrate (preferably 10, 15, 20 mM), 12-180 nM LDN-193189 or LDN-193189 2HCl (preferably 20, 50, 70, 80, 90, 100, 110, 120, 150, 170nM), and 55nM-25µM LY2157299, RepSox, SB5253334 or TEW7179 (preferably 100nM, 300nM, 500nM, 700nM, 900nM , 1µM, 2.5µM, 5µM, 7.5µM, 10µM, 12.5µM, 15µM, 20µM or 22µM).
6. The induction medium of item 5, characterized in that a basal medium is formed by 50% (v/v) DMEM medium and 50% (v/v) neural basal medium, then the basal medium is supplemented with 1% (v/v) N2 supplement, 2% (v/v) GS21 supplement or B27 supplement, 0.5mM HEPES buffer, 20mM glycine-glutamine monohydrate, 100nM LDN-193189 or LDN-193189 2HCl, and LY2157299, RepSox, SB5253334 or TEW7179 selected from 5µM, 7.5 µM or 12.5µM.
7. The induction medium of item 5 or 6, wherein the induction medium is formed as follows: a basal medium formed by 50% (v/v) DMEM medium and 50% (v/v) neural basal medium, then supplemented with 1% (v/v) N2 supplement, 2% (v/v) GS21 supplement or B27 supplement, 0.5mM HEPES buffer, 20mM glycine-glutamine monohydrate, 100nM LDN-193189 or LDN-193189 2HCl, and LY2157299 selected from 5µM, 7.5 µM or 12.5µM; preferably, the sensory neuron cell induction medium is further supplemented with a serum replacement (such as serum albumin, transferrin, fatty acid), more preferably, the serum replacement is in purified form.
8. A sensory neuron cell medium, characterized in that the induction medium of any one of items 5-7, and is supplemented with 3-10µM SU5402 (preferably 5µM, 6µM, 7µM, 8µM, 9µM), 3-10µM DAPT (4µM, 5µM, 6µM, 7µM, 8µM, 9µM), 1-3µM CHIR99021 (preferably 1.3µM, 1.5µM, 1.7µM, 1.9µM, 2.0µM, 2.2µM, 2.5µM, 2.7µM) or 0.5nM-3µM CHIR98014 (preferably 5nM, 50nM, 100nM, 200nM, 300mM, 500mM, 700mM, 900mM, 1µM, 1.4µM, 1.8µM, 2.0µM, 2.5µM, 2.7µM), and 0.1-49ng/ml human-NT3 (preferably 1, 5, 10, 15, 20, 25, 30, 35, 40, 45 ng/ml), preferably, the sensory neuron cell medium is further supplemented with a serum replacement (e.g., serum albumin, transferrin, fatty acid), more preferably, the serum replacement is in purified form.
9. A method for inducing pluripotent stem cells to differentiate into sensory neuron cells, characterized in that the pluripotent stem cells are cultured in the induction medium of any one of items 5-7 to obtain sensory nerve precursor cells, and then the sensory nerve precursor cells are cultured in the sensory neuron cell medium of item 8 to obtain sensory neuron cells, preferably, the culture is performed in the presence of a basal membrane, more preferably, the basal membrane is composed of one or more of Matrigel (STEMCELL Technologies), Laminin and Vitronectin.
10. Sensory nerve precursor cells, which are prepared by culturing pluripotent stem cells by using the induction medium of any one of items 5-7.
11. Sensory neuron cells, which are prepared by culturing the sensory nerve precursor cells of item 10 by using the sensory neuron cell medium of item 8.
12. A method for inducing sensory neuron cells comprising, culturing pluripotent stem cells by the induction medium of any one of items 5-7 to obtain sensory nerve precursor cells, and then culturing the sensory nerve precursor cells by the sensory neuron cell medium of item 8.
13. The method of item 9 or 12, wherein the pluripotent stem cells are prepared from somatic cells by using a reprogramming medium, preferably, wherein the reprogramming medium consists of a DMEM-F12 basal medium and supplementary components, the supplementary components include 60 µg/mL-180 µg/mL L-ascorbic acid, 5.3 µmol/L-74 µmol/L hydrocortisone, 3 ng/mL-89 ng/mL sodium selenite, 8 µmol/L-23 µmol/L Optiferrin, 0.5 µmol/L-7.4 µmol/L retinyl acetate, 40 ng/mL-60 ng/mL plant-derived recombinant human basic growth factor, 8 µg /mL-12 µg/mL IGF, 0.2 µmol/L-0.6 µg/mL A-83, 2 µmol/L-6 µmol/L CHIR99021, and 100 µmol/L-450 µmol/L sodium butyrate.
14. The method of item 13, characterized in that the supplementary components include 70 µg/mL-90 µg/mL L-ascorbic acid, 40 µmol/L-60 µmol/L hydrocortisone, 10ng/mL-30ng/mL sodium selenite, 8µmol/L-12µmol/L Optiferrin, 3µmol/L-6µmol/L retinyl acetate, 45 ng/mL-55 ng/mL plant-derived recombinant human basic growth factor, 8 µg/mL-12 µg/mL IGF, 0.3 µmol/L-0.5 µg/mL A-83, 3 µmol/L-5 µmol/L CHIR99021 and 280 µmol/L-410 µmol/L sodium butyrate.
15. The method of item 13, characterized in that the supplementary components comprise 80 µg/mL L-ascorbic acid, 50 µmol/L hydrocortisone, 20 ng/mL sodium selenite, 10 µmol/L Optiferrin, 4 µmol/L retinyl acetate, 50 ng/mL plant-derived recombinant human basic growth factor, 10 µg/mL IGF, 0.4 µg/mL A-83, 4 µmol/L CHIR99021 and 400 µmol/L sodium butyrate.
16. The method of any one of items 12-15, wherein the culturing is performed in the presence of a basal membrane, preferably, the basal membrane is composed of one or more of Matrigel (STEMCELL Technologies), Laminin and Vitronectin.
17. The method of any one of items 13-15, wherein the somatic cells are human mesenchymal cells, human CD34+ cells or human fibroblasts (e.g. skin fibroblasts, lung fibroblasts, bladder fibroblasts, foreskin fibroblasts, uterine fibroblasts).
18. Use of the sensory neuron of item 11 in screening a medicine for treating or alleviating pain or preparing a model for screening a medicine for treating or alleviating pain.

In some embodiments, the preparation of human induced sensory neuron comprises the following steps:
Step 1: Human derived somatic cells are subjected to somatic reprogramming by a plasmid method to obtain induced pluripotent stem cells, which are then identified cytologically and biochemically.
Step 2: The induced pluripotent stem cells obtained in step 1 are cultured in an adherent monolayer in a serum-free sensory neuron induction medium that does not contain substances acting on BMP transduction pathway, but contains, for example, GS21 Cell culture supplements and amino acid hydrates; thereby obtaining neuron precursor cells. In a particular embodiment, the substances acting on BMP signal transduction pathway include one or more of the following proteins in any combination: BMP2, BMP4, BMP4, Smad1, Smad5, Smad8; wherein, the induced pluripotent stem cells are cultured adherently for 15 days by using the serum-free sensory neuron induction medium to obtain an adherent monolayer of sensory nerve precursor cells.
Step 3: The precursor cells in Step 2 are cultured adherently in a serum-free sensory neuron maturation medium that does not contain one or more substances acting on BMP transduction pathway, TGF transduction pathway, Notch transduction pathway or VEGFR pathways, but contains, for example, GS21 cell culture supplements, neurotrophic factors and amino acid hydrates; thereby obtaining mature sensory neuron cells expressing ion channel proteins and having electrophysiological activity. In a particular embodiment, the substances acting on BMP signal transduction pathway include one or more proteins independently selected from the group consisting of: BMP2, BMP4, BMP4, Smad1, Smad5, and Smad8; wherein the substances acting on TGF transduction pathway include one or more proteins independently selected from the group consisting of: Activin, TGF-beta, Nodal, Smad2, and Smad3; the substances acting on Notch transduction pathway include one or more proteins independently selected from the group consisting of: Notch, NICD, and r-Secretase; the substances acting on VEGFR transduction pathway include one or more proteins independently selected from the group consisting of: VEGFR2, FGFR1, and PDGF-Rβ. The neuron precursor cells are further cultured adherently for 15 days in a serum-free sensory neuron maturation medium to obtain an adherent monolayer of sensory neuron cells, which express ion channel proteins and have electrophysiological activities.

In a particular embodiment, the adherent culture in steps 1 to 3 is preferably performed in the presence of a basal membrane. The basal membrane of the present invention can form a thin film composed of extracellular matrix molecules on the surface of culture dishes, and can provide support similar to the *in vivo* environment for parameters such as cell morphology, growth and differentiation, and movement. In a particular embodiment, the basal membrane is composed of one or more of Matrigel (STEMCELL Technologies), Laminin and Vitronectin.

The serum-free medium in the present invention means that it does not contain serum directly separated from blood. Serum is the clear liquid portion of plasma that does not contain fibrinogen or blood cells and remains liquid after the blood clots. The serum-free medium may contain serum replacements. Examples of serum replacements include purified substances such as serum albumin, transferrin, fatty acids, etc., which are substances well known in the art that can substitute for serum.

In the present invention, the expressions "sensory neuron cell" and "sensory neuron" may be used interchangeably.

### BRIEF DESCRIPTION OF THE DRAWINGS

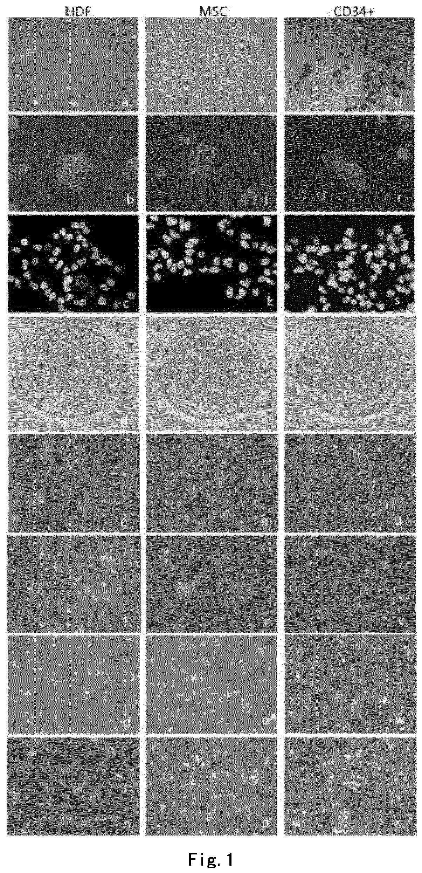

Figure 1: Identification of induced pluripotent stem cells derived from three types of cells, and sensory neurons obtained by using the present method and a control method. Figures 1a-1h show the results of peripheral neuron induction by using fibroblasts, wherein, Figure 1a shows fibroblasts (HDF) before reprogramming; Figure 1b shows iPSC clones formed by fibroblast reprogramming; Figure 1c shows Oct3/4 immunofluorescence identification of iPSC cells formed by fibroblast reprogramming; Figure 1d shows identification of the alkaline phosphatase of iPSC clones formed by fibroblast reprogramming; Figure 1e shows the peripheral sensory nerve precursor cells induced from fibroblasts with N2GS212I basal medium formula 2; Figure 1f shows the peripheral sensory neurons formed by fibroblasts by using the induction method of the present invention; Figure 1g shows the peripheral sensory nerve precursor cells formed by fibroblasts by using the serum induction method as a control; Figure 1h shows the peripheral sensory neurons formed by fibroblasts by using the serum induction method as a control; Figures 1i-Figure 1p show the results of peripheral neuron induction by using mesenchymal cells, wherein, Figure 1i shows mesenchymal cells before reprogramming; Figure 1b shows iPSC clones formed by reprogramming of mesenchymal cells; Figure 1k shows Oct3/4 immunofluorescence identification of iPSC cells formed by reprogramming of mesenchymal cells; Figure 1l shows alkaline phosphatase identification of iPSC clones formed by reprogramming of mesenchymal cells; Figure 1m shows the peripheral sensory nerve precursor cells induced from mesenchymal cells with N2GS212I basal medium formula 2; Figure 1n shows the peripheral sensory neurons formed from mesenchymal cells by using the induction method of the present invention; Figure 1o shows the peripheral sensory nerve precursor cells formed from mesenchymal cells by using the serum induction method as a control; Figure 1p shows the peripheral sensory neurons formed from mesenchymal cells by using the serum induction method as a control; Figure 1q-Figure 1x show data for peripheral neuron induction by using CD34+ cells, wherein, Figure 1q shows CD34+ cells before reprogramming; Figure 1r shows iPSC clones formed by reprogramming of CD34+ cells; Figure 1s shows Oct3/4 immunofluorescence identification of iPSC clones formed by reprogramming of CD34+ cells; Figure 1t shows alkaline phosphatase identification of iPSC clones formed by reprogramming of CD34+ cells; Figure 1u shows the peripheral sensory nerve precursor cells induced from CD34+ cells with N2GS212I basal medium formula 2; Figure 1v shows the peripheral sensory neurons formed from CD34+ cells by using the induction method of the present invention; Figure 1w shows the peripheral sensory nerve precursor cells formed from CD34+ cells by using the serum induction method as a control; Figure 1x shows the peripheral sensory neurons formed from CD34+ cells by using the serum induction method as a control.

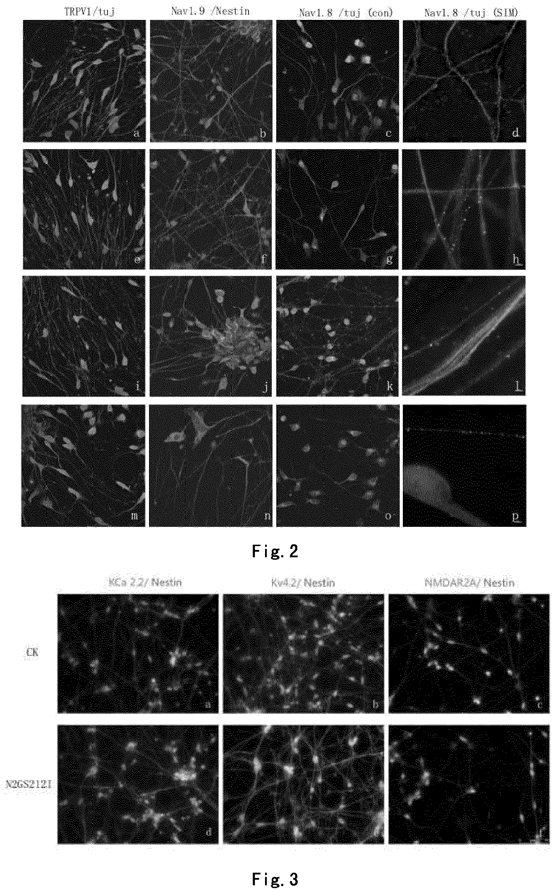

Figure 2: Immunofluorescence identification of the expression of sensory neuron-specific molecular marker. Figure 2a shows the immunofluorescence staining identification of the sensory neurons formed from CD34+ cell-derived induced pluripotent stem cells which sensory neurons have TRPV1/Tuj double positive expression; Figure 2b shows the immunofluorescence staining identification of the sensory neurons formed from CD34+ cell-derived induced pluripotent stem cells which sensory neurons express sodium ion channel 1.9 and Nestin; Figure 2c shows the immunofluorescence staining identification of the sensory neurons formed from CD34+ cell-derived induced pluripotent stem cells which sensory neurons have sodium ion channel 1.8/Tuj double positive expression; Figure 2d shows the distribution of the sensory neurons formed from CD34+ cell-derived induced pluripotent stem cells which sensory neurons have sodium ion channel 1.8/Tuj double positive expression under a high-resolution microscope; Figure 2e shows the immunofluorescence staining identification of the sensory neurons formed from fibroblast-derived induced pluripotent stem cells which sensory neurons have TRPV1/Tuj double positive expression; Figure 2f shows the immunofluorescence staining identification of the sensory neurons formed by fibroblast-derived induced pluripotent stem cells which sensory neurons have sodium ion channel 1.9/Nestin double positive expression; Figure 2g shows the immunofluorescence staining identification of the sensory neurons formed from fibroblast-derived induced pluripotent stem cells which sensory neurons have sodium ion channel 1.8/Tuj double positive expression ; Figure 2h shows the distribution of the sensory neurons formed by fibroblast-derived induced pluripotent stem cells which sensory neurons have sodium ion channel 1.8 and Tuj expression under a high-resolution microscope; Figure 2i shows the immunofluorescence staining identification of the sensory neurons formed by mesenchymal cell-derived induced pluripotent stem cells which sensory neurons have TRPV1/Tuj double positive expression ; Figure 2j shows the immunofluorescence staining identification of the sensory neurons formed by mesenchymal cell-derived induced pluripotent stem cells which sensory neurons have sodium ion channel 1.9/Nestin double positive expression ; Figure 2k shows the immunofluorescence staining identification of the sensory neurons formed by mesenchymal cell-derived induced pluripotent stem cells which sensory neurons have sodium ion channel 1.8/Tuj double positive expression ; Figure 2l shows the distribution of the sensory neurons formed from mesenchymal cell-derived induced pluripotent stem cells which sensory neurons have sodium ion channel 1.8 and Tuj expression under high-resolution microscopy ; Figure 2m shows the immunofluorescence staining identification of the sensory neurons formed from CD34+ cell-derived induced pluripotent stem cells by using the control method (i.e., serum induction method) which sensory neurons have TRPV1/Tuj double positive expression ; Figure 2n shows the immunofluorescence staining identification of the sensory neurons formed from CD34+ cell-derived induced pluripotent stem cells by using the control method (i.e., serum induction method) which sensory neurons have sodium ion channel 1.9 and Nestin expression; Figure 2o shows the immunofluorescence staining identification of the sensory neurons formed by CD34+ cell-derived induced pluripotent stem cells by using the control method (i.e., serum induction method) which sensory neurons have sodium ion channel 1.8/Tuj double positive expression ; Figure 2p shows the distribution of the sensory neurons formed from CD34+ cell-derived induced pluripotent stem cells by using the control method (i.e., serum induction method) which sensory neurons have sodium ion channel 1.8 and Tuj expression under high-resolution microscopy .
Figure 3: The difference in the expression and localization of various ion channel proteins and the expression of sensory neuron ion channels and receptor markers are compared between the method of the present invention (N2GS212I) and the control method of serum induction method (CK) by immunofluorescence. Taking CD34+-derived iPSCs as an example, Figure 3a shows the expression and localization of potassium-calcium complex ion channel 2.2 in peripheral neurons induced by the control method; Figure 3b shows the expression and localization of potassium ion channel 4.2 in peripheral neurons induced by the control method; Figure 3c shows the expression and localization of NMDAR2A in peripheral neurons induced by the control method; Figure 3d shows the expression and localization of potassium-calcium complex ion channel 2.2 in peripheral neurons induced by the method of the present invention; Figure 3e shows the expression and localization of potassium ion channel 4.2 in peripheral neurons induced by the method of the present invention; Figure 3f shows the expression and localization of NMDAR2A in peripheral neurons induced by the method of the present invention.

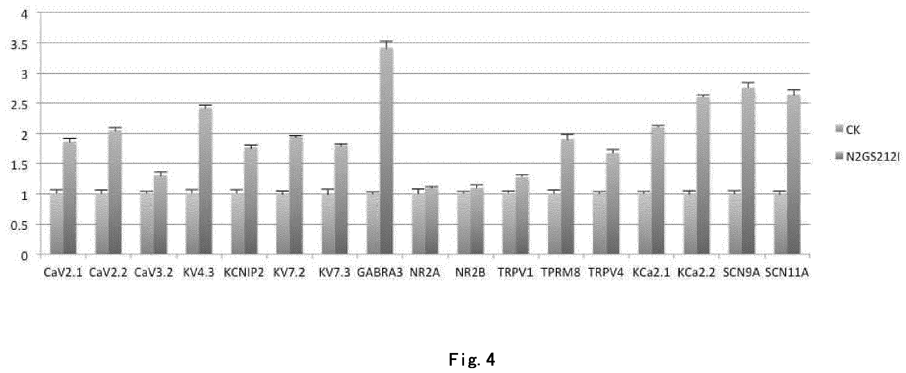

Figure 4: The differences in the expression of various ion channel genes are compared between the method of the present invention (N2GS212I) and the control method of serum induction method (CK) by Q-PCR, by taking sensory neurons derived from CD34+ cells as an example. As compared with the serum control induction method, the expressions of various ion channels in the peripheral neurons obtained by the present method are significantly increased compared with the control group.

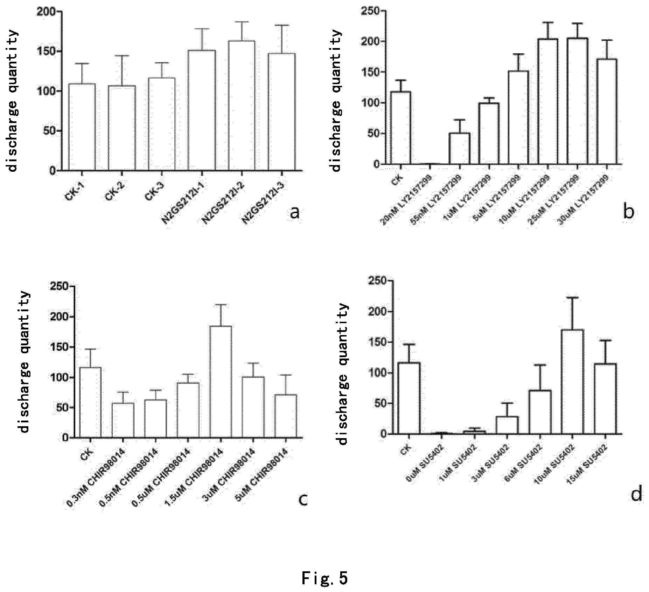

Figure 5: The difference in electrophysiological activity between peripheral neurons prepared by the method of the present invention and peripheral neurons prepared by the control serum induction method is compared by multi-electrode array (MEA). Figure 5a shows the comparison of electrophysiological activity of cells obtained from different cell sources and different induction methods. CK represents the sensory neurons prepared with the serum induction control method; wherein, CK-1 represents the sensory neurons formed by the CD34+ cell-derived induced pluripotent stem cells; CK-2 represents the sensory neurons formed by fibroblast-derived induced pluripotent stem cells; CK-3 represents the sensory neurons formed by mesenchymal cell-derived induced pluripotent stem cells. N2GS212I represents the sensory neurons prepared by the method in Example 3 of the present invention; wherein, N2GS212I-1 represents the sensory neurons formed by the CD34+ cell-derived induced pluripotent stem cells; N2GS212I-2 represents the sensory neurons formed by fibroblast-derived induced pluripotent stem cells; N2GS212I-3 represents the sensory neurons formed by mesenchymal cell-derived induced pluripotent stem cells. 5b represents the comparison of electrophysiological activities of cells induced by different concentrations of LY2157299, taking mesenchymal cell-derived sensory neurons as an example; 5c represents the comparison of electrophysiological activities of cells induced by different concentrations of CHIR98014, taking mesenchymal cell-derived sensory neurons as an example; 5d represents the comparison of electrophysiological activities of cells induced by different concentrations of SU5402, taking mesenchymal cell-derived sensory neurons as an example.

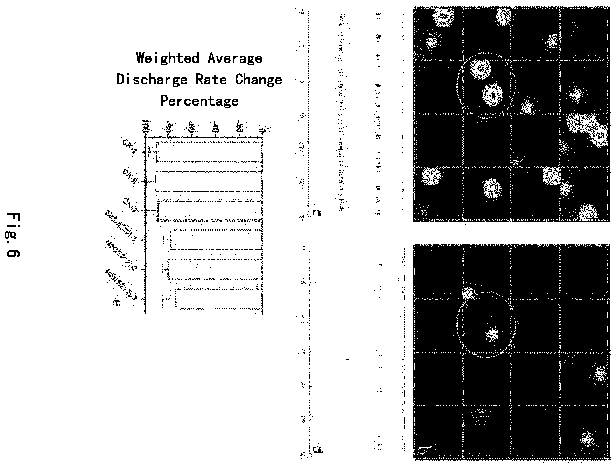

Figure 6: Inhibition of electrical signal of sensory neurons by sodium ion channel inhibitors is validated by multi-electrode array (MEA) at neuron maturation stage (20 days after induction). Figures 6a and 6c show the heat map of spontaneous discharge frequency (6a) and 30-second recording of single-hole discharge frequency (6c) of sensory neurons cultured at 37°C and 5% carbon dioxide; Figures 6b and 6d show the heat map of spontaneous discharge frequency (6b) and 30-second recording of single-hole firing frequency (6d) of sensory neurons cultured at 37°C and 5% carbon dioxide after adding 3µM tetrodotoxin to culture system to block the sodium ion channel; Figure 6e shows the percent inhibition of peripheral neuronal potential after adding 3µM tetrodotoxin to inhibit the sodium ion channel, as compared with that before adding 3µM tetrodotoxin under the same conditions. CK represents the sensory neurons prepared with the serum induction method; wherein, CK-1 represents the sensory neurons formed by the CD34+ cell-derived induced pluripotent stem cells; CK-2 represents the sensory neurons formed by fibroblast-derived induced pluripotent stem cells; CK-3 represents the sensory neurons formed by mesenchymal cell-derived induced pluripotent stem cells. N2GS212I represents the sensory neurons prepared by the method in Example 3; wherein, N2GS212I-1 represents the sensory neurons formed by the CD34+ cell-derived induced pluripotent stem cells; N2GS212I-2 represents the sensory neurons formed by fibroblast-derived induced pluripotent stem cells; N2GS212I-3 represents the sensory neurons formed by mesenchymal cell-derived induced pluripotent stem cells.

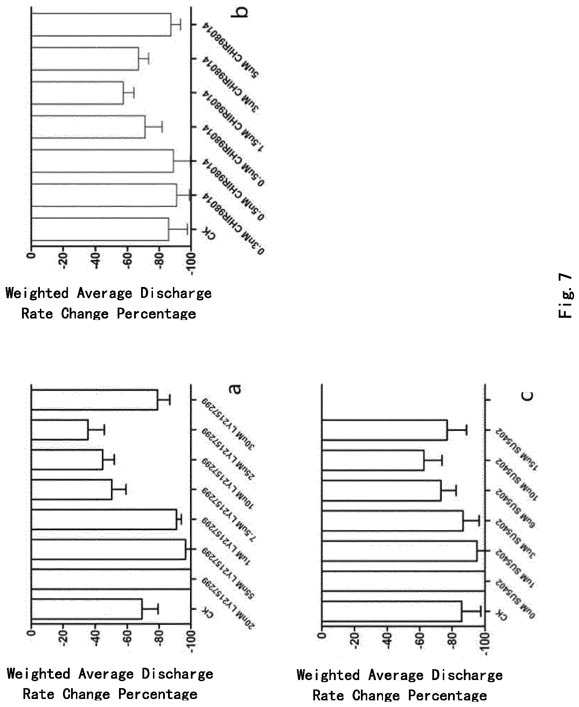

Figure 7: Verification of decline percentage in peripheral neuronal potential at neuron maturation stage (after 20 days of induction) by multi-electrode array (MEA) after addition of 1 µM PF-05089771 to specifically inhibit sodium ion channel 1.7 as compared to before addition of 1 µM PF-05089771. Figure 7a shows the decline percentage of potential in cells induced by various LY2157299 concentrations after using 1µM PF-05089771, taking fibroblast-derived sensory neurons as an example; Figure 7b shows the decline percentage of potential in cells induced by various CHIR98014 concentrations after using 1µM PF-05089771, taking fibroblast-derived sensory neurons as an example; Figure 7c shows the decline percentage of potential in cells induced by various SU5402 concentrations after using 1µM PF-05089771, taking fibroblast-derived sensory neurons as an example.

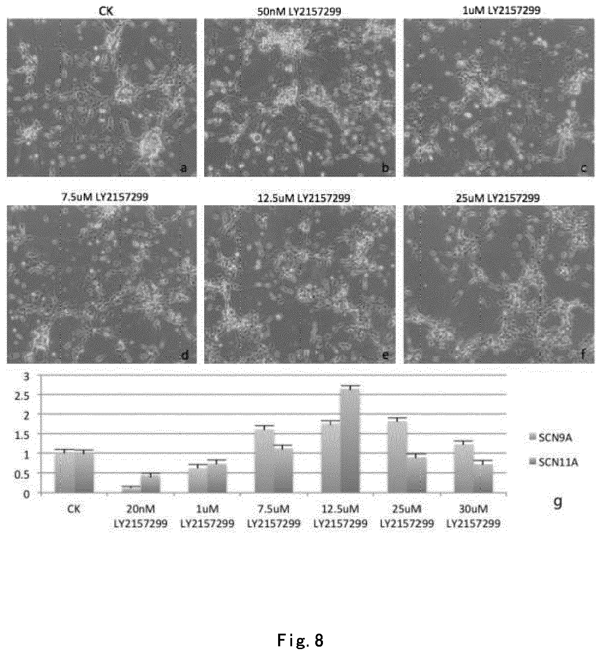

Figure 8: Morphology of peripheral sensory neuron cell induced by different LY2157299 concentrations and difference in sodium ion channel expression thereof. Figure 8a shows cells obtained by serum induction method; Figures 8b-8f show the neurons obtained by different concentrations of LY2157299 (50nM-25µM). Figure 8g shows comparison of expressions of different sodium ion channel proteins in peripheral neurons under different concentrations of small molecule LY2157299 via Q-PCR.

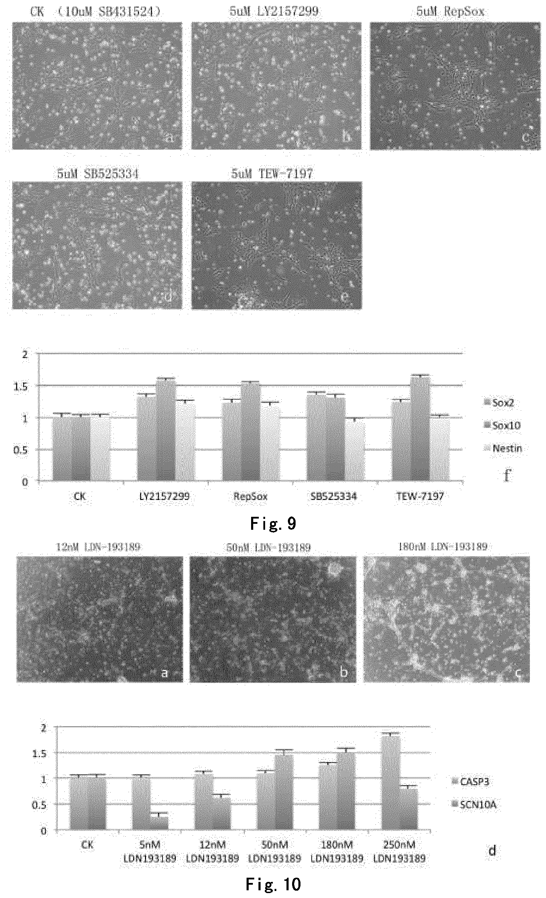

Figure 9: Morphology of neuron precursor cells induced by different inhibitor molecules and differences in expression of precursor cell markers. Figure 9a shows neuron precursor cells formed by serum induction method (10µM SB431524); Figure 9b shows neuron precursor cells formed by using 5µM LY2157299; Figure 9c shows neuron precursor cells formed by using 5µM RepSox; Figure 9d shows neuron precursor cells formed by using 5µM SB525334; Figure 9e shows neuron precursor cells formed by using 5µM TEW-7197; Figure 9f shows differential expressions of neuron precursor cell markers during neuron precursor cell formation by Q-PCR between the method of using small molecule inhibitors of the present invention and the control serum induction method (CK).
Figure 10: Effects of different LDN concentrations on the formation of peripheral sensory neuron cells. Figures 10a-10c show that cell types with neural axons can be induced only at low (12nM), medium (50nM) and high (180nM) concentrations of LDN193189. Figure 10d shows the differential expressions of peripheral sensory neuron genes resulting from different concentrations of LDN193189 by Q-PCR.

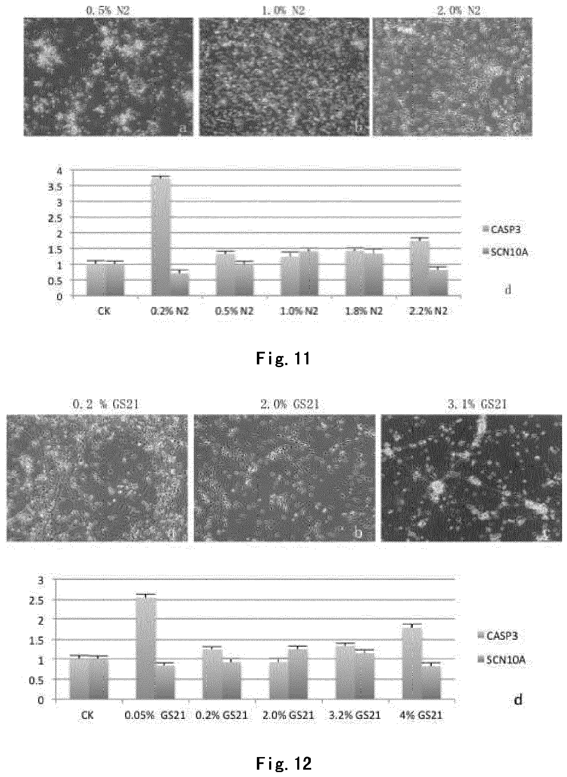

Figure 11: Effects of different N2 concentrations on the formation of peripheral sensory cells. Figures 11a-11c show that cell types with neural axons can be obtained only at low (0.5%), medium (1.0%) and high (1.8%) concentrations of N2. Figure 11d shows the differential expressions of peripheral sensory neuron cell genes resulting from different concentrations of N2 by Q-PCR.
Figure 12: Effects of different GS21 concentrations on the formation of peripheral sensory neuron cells. Figures 12a-12c show that cell types with neural axons can be obtained only at low (0.2%), medium (2.0%) and high (3.1%) concentrations of GS21. Figure 12d shows the differential expressions of peripheral sensory neuron cell genes resulting from different concentrations of GS21 by Q-PCR.

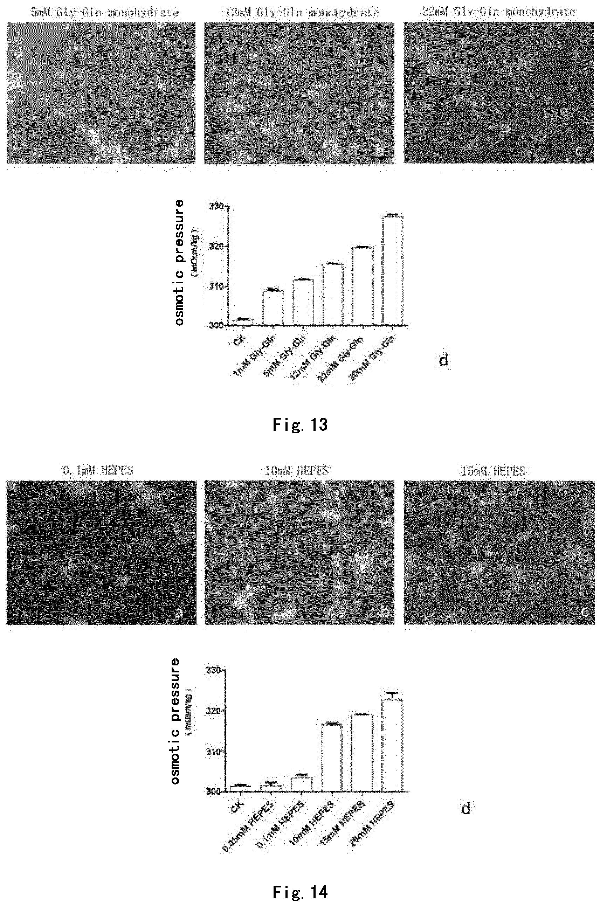

Figure 13: Effects of different concentrations of glycine-glutamine monohydrate on the formation of peripheral sensory cells. Figures 13a-13c: The induced peripheral neurons prepared by using low (5 mM), medium (12 mM) and high (22 mM) concentrations of glycine-glutamine monohydrate have a normal morphology, but have a slow axonal growth at high concentration (22 mM). Figure 13d represents the osmotic pressure data for the medium with different concentrations of glycine-glutamine monohydrate.
Figure 14: Effects of different HEPES buffer concentrations on the formation of peripheral sensory neuron cells. Figures 14a-14c show that at low concentration (0.1 mM), medium concentration (10 mM) and high concentration (15 mM) of HEPES buffer, the cells are morphologically normal, and cell intolerance begins to appear at high concentration (15 mM). Figure 14d shows the osmotic pressure data for the medium with different HEPES buffer concentrations.

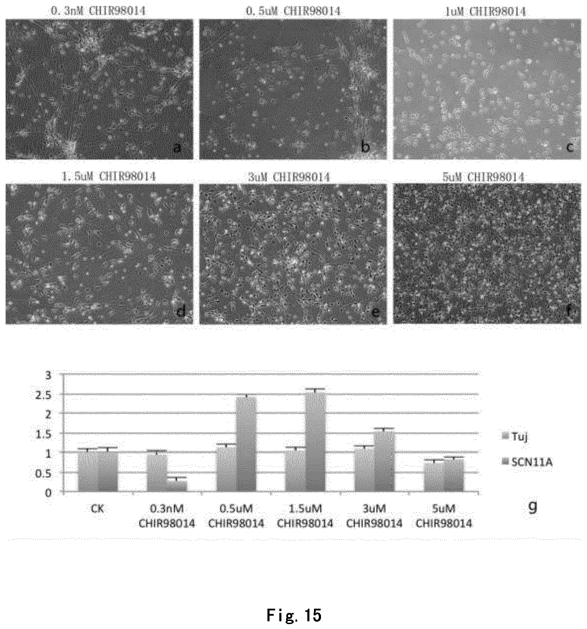

Figure 15: Effects of different CHIR98014 concentrations on the formation of peripheral sensory cells. Figures 15a-15f show the neurons obtained by different concentrations of CHIR98014 (0.3nM-5µM). Figure 15g shows the effects of different concentrations of CHIR98014 on the expressions of peripheral sensory neuron-specific genes by using Q-PCR analysis.

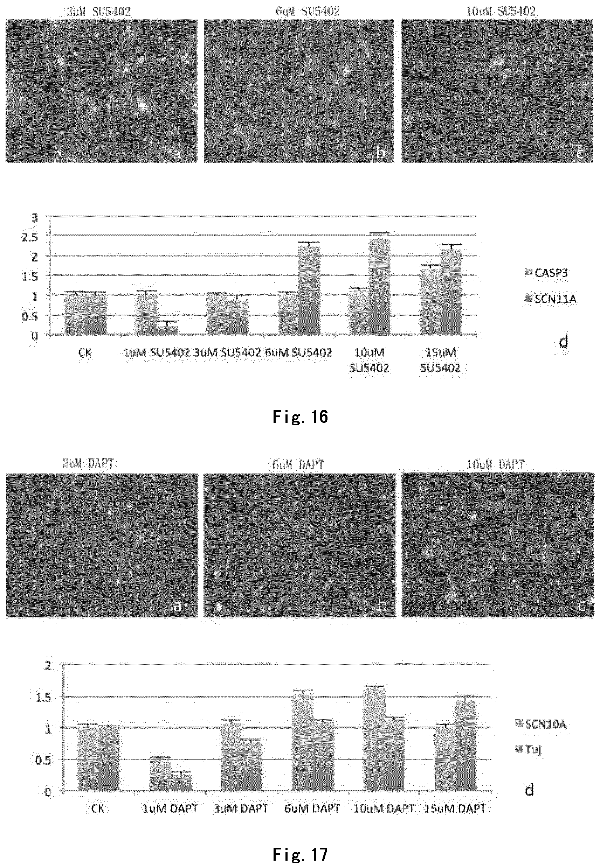

Figure 16: Effects of different SU5402 concentrations on the formation of peripheral sensory neuron cells. Figures 16a-16c show that cells can form axons at low concentration (3µM), medium concentration (6µM) and high concentration (10µM) of SU5402; Figure 16d shows the effects of different concentrations of SU5402 on the expressions of peripheral sensory neuron cell-specific genes by using Q-PCR analysis.
Figure 17: Effects of different DAPT concentrations on the formation of peripheral sensory neuron cells. Figures 17a-17c show that cells are able to form axons at low (3µM), medium (6µM) and high (10µM) concentrations of DAPT. Figure 17d show the effects of different concentrations of DAPT on the expressions of peripheral sensory neuron cell-specific genes by using Q-PCR analysis.

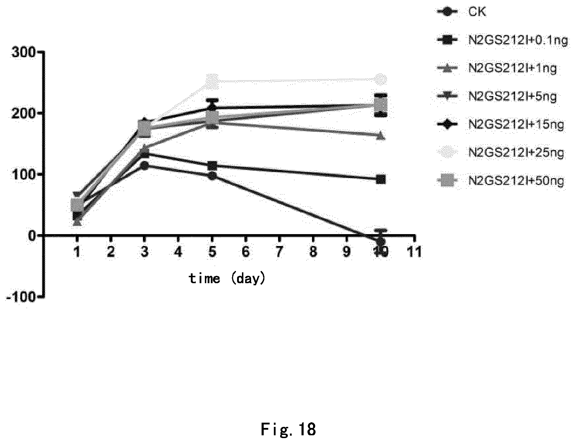

Figure 18: Quantitative detection of cell viability by Cyquant assay.

### Benefitial effect

The present invention provides a new combination of chemical molecules, which can significantly improve the purity of induced sensory neurons *in vitro* and the expression intensity of functional ion channel proteins, and the formula of the present invention can support *in vitro* culture of induced peripheral neuron cells, thereby establishing a sensory neuron cell induction culture system without serum and animal-derived substances. Therefore, sensory neuron cells are obtained by reprogramming and directional differentiation of somatic cells from various sources, and they have various complete biological functions. The state is stable in multiple batches, and the reproducibility is good, which can solve the problem that sensory neuron cells are not easy to obtain in medicine screening and scientific research. The invention also solves the long-standing problems involving animal-derived culture methods and feeder cell contamination, and can be used for *in vitro* screening of medicines for neurological diseases, especially analgesic medicines, and has great economic and social effects.

### DETAILED DESCRIPTION OF THE INVENTION

The implementation process and beneficial effects of the present invention are described in detail below through specific examples, which are intended to help readers better understand the essence and characteristics of the present invention, and are not intended to limit the scope of the present application.

### Example 1 Preparation of human induced pluripotent stem cells

A 6-well culture plate was coated with Matrigel (STEMCELL Technologies), and then incubated in a 37°C incubator for more than one hour. The following human somatic cells were reprogrammed to induced pluripotent stem cells by using a Neon Electrotransformation Kit (Thermo Fisher) when somatic cell reprogramming was performed using the Epi5 Reprogramming Kit (Invitrogen): Human Mesenchymal Cells (Lonza, PT-2501) , human CD34+ cells (PromoCELL, c-12921), and human skin fibroblasts (PromoCELL, c-12302).

The steps were as follows: after centrifugation and washing, the human somatic cells were resuspended in a resuspension buffer provided in the electrotransformation kit, and the electrotransformation was performed according to the following procedures: 1650V pulse voltage, 10ms pulse width, 3 pulses. After electrotransformation, the cells were removed into a 6 ml tube of reprogramming medium, mixed well, and added to each well of a 6-well plate. The conditions for cell culture were 37°C, 5% CO₂ (Panasonic, MCO-18AC), and the cells were cultured by standing still. Thereafter, the medium was half quantity changed a day until the tenth day after electrotransformation, and the medium was changed every other day from the tenth day until the 28th day.

The medium and method used in the reprogramming process were implemented with reference to the patent "Reprogramming Medium and Culture Method for Reprogramming Induced Pluripotent Stem Cell" (ZL 201910050800.7). The reprogramming medium consisted of DMEM-F 12 basal medium and supplementary components, wherein the supplementary components are 80µg/ml ascorbic acid, 50µmol/L hydrocortisone, 20ng/ml sodium selenite, 10µmol/L Optiferrin, 0.4µg/ml IGF, 0.4µg/ml A-83, 4µmol/L CHIR9901 and 400µmol/L sodium butyrate.

### Example 2. Counting of induced pluripotent stem cell clones and identification of pluripotency

(2.1) The counting of induced pluripotent stem cell clones and identification of pluripotency were performed by using alkaline phosphatase staining (Cat# SCR004, Millipore). Firstly, the cell culture medium in the petri dish was aspirated, and the cells were washed with PBS, fixed for 1-2 min by adding 4% paraformaldehyde, then washed with TBST for 3 times, added with staining working solution provided in the kit (taking 24-well plate as an example, 0.5 ml per well), and placed at room temperature in the dark for 15-20 min; after staining, the staining solution was aspirated, and the cells were washed with phosphate buffer for 2-3 times, and the staining results were observed by a microscope (DMi8, Leica).

(2.2) Oct3/4 immunofluorescence identification of induced pluripotent stem cells by using immunofluorescence.

The induced pluripotent stem cells in Example 1 were respectively taken and identified by immunofluorescence staining as follows: cells were fixed with 4% paraformaldehyde at room temperature for 20 minutes, washed twice with DPBS buffer; then treated with 0.1% Triton X-100 for 50 minutes, washed twice with DPBS buffer; then incubated overnight at 4°C with DPBS buffer containing 10% horse serum and 0.1% Triton X-100; added with primary antibody diluted in DPBS buffer, incubated at 37°C for 2 hours, washed three times with DPBS buffer; then added with corresponding secondary antibody diluted in DPBS buffer, incubated at 37°C for 2 hours, washed three times with DPBS buffer; and then being subjected to nuclear staining with 0.1µg/ml DAPI solution for 2 minutes, washed with DPBS buffer for three times, and then photographed (DMi8, Leica). The details for the antibodies used were shown in Table 1.

**Table 1. Antibodies used for immunofluorescence staining of pluripotent stem cells**

| Primary Antibody | Primary Antibody Concentration | Secondary Antibody | Secondary Antibody Concentration |
|---|---|---|---|
| Anti-Oct4 antibody (CST23) | 1:200 | Goat anti-rabbit IgG(H+L), Alexa Fluor 488 (Invitrogen A11008) | 1:1000 |

The results for alkaline phosphatase staining and immunofluorescence staining were shown in Figure 1, which showed changes before and after induction, alkaline phosphatase staining results and Oct3/4 immunofluorescence identification results in Figures 1a, 1b, 1c, and 1d (fibroblasts), Figures 1i, 1j, 1k, and 1l (mesenchymal cells), and Figures 1q, 1r, 1s , and 1t (CD34+ cells).

### Example 3. Sensory neuron differentiation of induced pluripotent stem cells

### (3.1) Induction of sensory nerve precursor cells

A T25 cell culture flask was coated with Matrigel (STEMCELL Technologies), and then incubated in a 37°C incubator for more than one hour. Human induced pluripotent stem cells from various sources obtained in Example 1 were seeded in a T25 culture flask at 1×10⁶ cells.

When the induced pluripotent stem cells reached 70% coverage, they were digested with 0.05% trypsin/EDTA for 5 min at 37°C, and the cell digestion was terminated by using DMEM. After the cells were washed and centrifuged, they were re-seeded in a T25 culture plate at 2 × 10⁵ per flask, and cultured in a sensory neuron induction medium at 37°C, 5% CO₂ (Panasonic, MCO-18AC) for 10 days until the formation of neural plate, which confirmed the formation of sensory nerve precursor cells.

The results of three different cells cultured in the sensory neuron induction medium were shown in Figures 1e, 1m and 1u.

Formula for sensory neuron induction medium (N2GS212I-1) was as follows:
50% (v/v) DMEM medium and 50% (v/v) Neurobasal medium formed a basal medium, which was supplemented with 1% (v/v) N2 supplement, 2 % (v/v) GS21 supplement, 0.5 mM HEPES buffer, 20 mM glycine-glutamine monohydrate, 100 nM LDN-193189, and 7.5 µM LY2157299.

### (3.2) Induction of sensory nerve precursor cells by using a medium containing serum components (represented by CK, also referred to as "serum induction method" in the present invention)

According to published method (Nat Biotechnol., 2013, 30(7): 715-720), three types of cells (fibroblasts, mesenchymal cells, and CD34+ cells) were induced to sensory nerve precursor cells by using a serum-containing media. The specific steps were as follows: The basal medium was prepared by 820ml Knockout Duchenne's modified Eagle's medium (Knockout DMEM medium, Cat. No. 10829018, Thermo Fisher), 150ml Knockout Serum Replacement (Cat. No. 10828028, Thermo Fisher), 1mM L-Glutamine (Cat. No. 25030081, Thermo Fisher), 100µM Minimum Essential Medium Non-Essential Amino Acids (Cat. No. 11140076, Thermo Fisher) and 0.1mM β-mercaptoethanol (Cat. No. 21985023, Thermo Fisher), and the basal medium was supplemented with 100 nM LDN-193189 and 10 µM SB431542 on Day 0 to Day 5. N2 medium was formed by adding 1% (v/v) N2 supplement (Cat. No. 17502045, Thermo Fisher) to neural basal medium (Neurobasal Medium, Cat. No. 21103049, Thermo Fisher). N2 medium was added every other day starting from day 4 in 25% increments (100% N2 on day 10). On day 2 to day 10, three inhibitors, 3µM CHIR99021 (Selleck, Cat. No. S2924), 10µM SU5402 (Tocris, Cat. No. 3300/1) and 10µM DAPT (Selleck, Cat. No. S2215), were added to induce sensory nerve precursor cells- CK. The results were shown in Figures 1g (CK-fibroblasts), 1o (CK-mesenchymal cells) and 1w (CK-CD34+ cells).

### (3.3) Culture of sensory neuron cells

After obtaining the above-mentioned various sensory nerve precursor cells, the medium was removed, and cells were washed with DPBS, and then treated with 0.05% trypsin/EDTA at 37°C for 5 minutes, and then cell digestion was terminated with DMEM. After the cells were washed and centrifuged, the cells were re-seeded in a T25 culture flask at 1-5×10⁵ per plate, and the sensory nerve precursor cells were induced to differentiate and expand into mature sensory neurons by using sensory neuron cell medium. The conditions for cell culture were 37°C, 5% CO₂. The results were shown in Figures 1f (fibroblasts) and 1h (CK-fibroblasts), 1n (mesenchymal cells) and 1p (CK-mesenchymal cells), 1v (CD34+ cells) and 1x (CK-CD34+ cells).

Sensory Neuron Cell Medium was prepared as follows: 10 µM SU5402 (Tocris, Cat. No. 3300/1), 10µM DAPT (Selleck, Cat. No. S2714), 4.9µM CHIR98014 (Selleck, Cat. No. S2745); 49ng/ml human-NT3 (Novoprotein, Cat. No. C079) were added into Sensory Neuron Induction Medium N2GS212I-1.

### Example 4. Identification of sensory neuron cells

### (4.1) Identification of molecular markers specific for sensory neurons

Induced sensory neurons were identified for marker expression by using immunofluorescence (immunofluorescence staining of multiple markers for different cell types). The various sensory neuron cells in Example 3 were respectively taken, and identified by immunofluorescence staining according to section (2.2) of Example 2. Details for antibodies used were shown in Table 2.

**Table 2. Antibodies used for immunofluorescence staining of peripheral sensory neuron cells**

| Primary Antibody | Primary Antibody Concentration | Secondary Antibody | Secondary Antibody Concentration |
|---|---|---|---|
| Anti-Nav1.9 antibody (ab65160) | 1:150 | Goat anti-rabbit IgG(H+L), Alexa Fluor 488 (Invitrogen A11008) | 1:1000 |
| Anti-NESTIN antibody (MAB5326) | 1:200 | Donkey anti-mouse IgG(H+L), Alexa Fluor 568 (Invitrogen A11008) | 1:1000 |
| Anti-TRPV1 antibody (NHA12699) | 1:500 | Goat anti-rabbit IgG(H+L), Alexa Fluor 488 (Invitrogen A10037) | 1:1000 |
| Anti-Nav 1.8 antibody (NHA11324) | 1:250 | Goat anti-rabbit IgG(H+L),Alexa Fluor 488 (Invitrogen A11008) | 1:1000 |
| Anti-Tuj antibody (MAB 1637) | 1:150 | Donkey anti-mouse IgG(H+L),Alexa Fluor 568 (Invitrogen A11008) | 1:1000 |

The immunofluorescence staining results of various sensory neuron cells obtained in Example 3 were shown in Figures 2 and 3 . The results showed that in present invention, a variety of somatic cells can be successfully induced into sensory neuron cells, expressing a series of sensory neuron-specific markers, and showing a higher level of marker expression compared with the serum induction method.

Figures 2a-21 illustrate sensory neurons formed by using induced pluripotent stem cells from different sources, wherein the sensory neuron cells induced by the sensory neuron induction medium of the present invention had no significant difference in ion channel expression.

The sensory neurons produced by serum-induction of iPSCs from mesenchymal cells and fibroblasts were similar to those in Figures 2m-2p (CD34+ cells as an example). Figures 2o and 2p showed the results of serum induction method, wherein as compared with the small molecule induction method of the present invention, the expression of sodium ion channel 1.8 produced by sensory neuron cells in serum induction method was weaker, and channel proteins were unevenly distributed in cell structure.

### (4.2) Immunofluorescence identification of the expression and localization of various ion channel proteins in sensory neuron cells

Sensory neuron cells were identified for marker expression by using immunofluorescence. Taking the sensory neuron cells differentiated from iPSCs induced by CD34+ source of Example 3 as an example, immunofluorescence staining was carried out according to the description in section (2.2) of Example 2. The details for the antibodies used were shown in Table 3.

**Table 3. Antibodies used for immunofluorescence staining of peripheral neuron markers**

| Primary Antibody | Primary Antibody Concentration | Secondary Antibody | Secondary Antibody Concentration |
|---|---|---|---|
| Anti-NESTIN antibody (MAB5326) | 1:200 | Donkey anti-mouse IgG(H+L),Alexa Fluor 568 (Invitrogen A11008) | 1:1000 |
| Anti-KCa2.2 antibody(APC-028) | 1:50 | Goat anti-rabbit IgG(H+L),Alexa Fluor 488 (Invitrogen A10037) | 1:1000 |
| Anti-KV4.2 antibody(APC-023) | 1:50 | Goat anti-rabbit IgG(H+L),Alexa Fluor 488 (Invitrogen A10037) | 1:1000 |
| Anti-NMDA | 1:50 | Goat anti-rabbit | 1:1000 |
| Receptor 2A Antibody (CST4205) | | IgG(H+L),Alexa Fluor 488 (Invitrogen A10037) | |

The results were shown in Figure 3. In Figure 3, the potassium-calcium complex ion channel 2.2 and potassium ion channel 4.2 in the peripheral sensory neuron cells induced in the present invention had more uniform distribution and abundance than the control group (serum induction method), while there was no significant difference in the expression of NMDAR2A. This result was consistent with Q-PCR (Figure 4) results. The images above could represent the results for mesenchymal and fibroblast-derived sensory neuron cells.

The results showed that in present invention, a variety of somatic cells can be successfully induced into sensory neuron cells, expressing a series of sensory neuron-specific markers, and showing a higher level of marker expression compared with the serum induction method.

### (4.3) Transcription changes of different marker genes during induction from pluripotent stem cells to sensory neurons were detected by Q-PCR.

Total RNA from different sensory neuron cells was extracted by RNeasy Mini or Micro Kit (QIAGEN), and cDNA was synthesized from 1 mg RNA by SuperScript III First-Strand Synthesis System (Invitrogen). SYBR Premix Ex Taq (TaKaRa) and Thermal Cycler Dice Real Time System (TaKaRa) were used for labeling and reaction of Quantitative PCR, and beta-Actin was used as internal control. All data were analyzed by delta-Ct method. Triplicates were used for each group of experiments, and statistics were performed with ANOVA. Primer sequences used to identify coding genes of different cellular markers were shown in Table 4.

**Table 4. Primers used in Q-PCR for peripheral neuron and pluripotent stem cell markers**

| | |
|---|---|
| SOX10-F | CCACCTATGCCACAGTGCCTAAG (SEQ ID NO: 1) |
| SOX10-R | GTGCCAACTCCTTCCTGCCTTC (SEQ ID NO:2) |
| SCN9A -F | AAGAGGATGTGTCTGCTACTGTCA (SEQ ID NO:3) |
| SCN9A-R | GAAGGTGGAGAGGTGGTGGATG (SEQ ID NO:4) |
| SCN10A-F | AACTTCCAGACCTTCGCCAACAG (SEQ ID NO:5) |
| SCN10A-R | TGGTGAAGAAGATGATGCCTACGG (SEQ ID NO:6) |
| SCN11A-F | TGATGATGTCGCTTCCTTCTCTGT (SEQ ID NO:7) |
| SCN11A-R | CCAACCTGCTGATGTGCTTATCTG (SEQ ID NO:8) |
| Nestin-F | TCAAGATGTCCCTCAGCCTGGA (SEQ ID NO:9) |
| Nestin-R | TGGCACAGGTGTCTCAAGGGTAG (SEQ ID NO:10) |
| CaV2.1-F1 | TGGAGGACGAGGACAGTGATGAA (SEQ ID NO:11) |
| CaV2.1-R1 | CGAGTCAGGATGCCAGAGTTCTT (SEQ ID NO:12) |
| Cav2.2-F1 | CAGCATCAACCGCCACAACAAC (SEQ ID NO:13) |
| Cav2.2-R1 | GGAGCACAGGAAGATGAAGGAGAC (SEQ ID NO:14) |
| CaV3.2-F1 | GCTGGTGGTGGAGACGCTGATA(SEQ ID NO:15) |
| CaV3.2-R1 | ACTGTGCCTTGGTGGAGATGTTC(SEQ ID NO:16) |
| KV4.3-F1 | CCTGCTGCTCCCGTCGTAGTAA (SEQ ID NO: 17) |
| KV4.3-R1 | GATGCTGATGATGGCTGTGGTGAT (SEQ ID NO:18) |
| KCNIP2-F2 | GCTCCAAGTTCCACAGGTCTGCTA (SEQ ID NO:19) |
| KCNIP2-R2 | CACCACAACCACCACCAACACAT (SEQ ID NO:20) |
| KV7.2-F2 | TGTATGTGTCCTTGTGCCGTAGTG (SEQ ID NO:21) |
| KV7.2-R2 | GCATCAACCTGTCCGTGTGAGTAA (SEQ ID NO:22) |
| KV7.3-F2 | GCATATTCAACCAAAGCCCGTGTA (SEQ ID NO:23) |
| KV7.3-R2 | AAGGACCTGCTGAACAGACAAGAG (SEQ ID NO:24) |
| GABRA3-F1 | GGACTGGTTCATAGCCGTCTGTT (SEQ ID NO:25) |
| GABRA3-R1 | ACGATGTTGAAGGTAGTGCTGGTT (SEQ ID NO:26) |
| TRPV4-F1 | TGCCGTGACAGCGAGACCTT (SEQ ID NO:27) |
| TRPV4-R1 | CAGATGTGCTTGCTCTCCTTGGA (SEQ ID NO:28) |
| TRPM8-F1 | GGCACCGTCCAGGAGAACAATG (SEQ ID NO:29) |
| TRPM8-R1 | GCAGCAACACTTGAAGCACTTCTT (SEQ ID NO:30) |
| KCa2.1-F2 | TGGCTGGAAGGCACTGGTGAT (SEQ ID NO:31) |
| KCa2.1-R2 | GCTGGTACAGGTGTCCCTAGAGAG (SEQ ID NO:32) |
| KCa2.2-F1 | GGAGTGAAGACTTCGAGAAGAGGA (SEQ ID NO:33) |
| KCa2.2-R1 | TGGTGGTGCTGTGGAAGAGGA (SEQ ID NO:34) |
| NR2A-F1 | GTTGGTGATGGTGAGATGGAGGAG (SEQ ID NO:35) |
| NR2A-R1 | CCAGATGAAGGTGATGAGGCTGAG (SEQ ID NO:36) |
| NR2B-F1 | ACTACTGCTGCTGCTGGTTCA (SEQ ID NO:37) |
| NR2B-R1 | AAGGTATGACTGAGTTGGCTGTGA (SEQ ID NO:38) |
| TRPV1-F1 | GCAGTGCCTTCTTCATCCTTCCTT (SEQ ID NO:39) |
| TRPV1-R1 | AAGCATGTGCCATTGCGGAGAA (SEQ ID NO:40) |
| CASP3-F | GGAAGCGAATCAATGGACTCTGG (SEQ ID NO:41) |
| CASP3-R | GCATCGACATCTGTACCAGACC (SEQ ID NO:42) |
| ACTIN-F | TCCCTGGAGAAGAGCTACGA (SEQ ID NO:43) |
| ACTIN-R | AGCACTGTGTTGGCGTACAG (SEQ ID NO:44) |

Taking the sensory neuron cells obtained from CD34+ source by the method of Example 3 and the sensory neuron cells CK obtained by serum induction method as examples, the results were shown in Figure 4 . Compared with the serum induction method, the sensory neuron cells obtained by the present invention had significant increase in the expressions of various important channel genes such as potassium ion channel genes (KV4.3, KCNIP2, KV7.2, KV7.3), calcium ion channel proteins (CaV2.1, CaV2.2, CaV3.2), potassium-calcium complex ion channels (KCa2.1, KCa2.2), sodium ion channels (SCN9A, SCN11A), aminobutyric acid (ABA) receptors (GABRA3), aspartate receptors (NR2A, NR2B), TRP ion channel family (TRPV1, TRPV4, TPRM8). The results showed that in present invention, a variety of somatic cells can be successfully induced into sensory neuron cells, expressing a series of sensory neuron-specific markers, and showing a higher level of marker expression compared with the sensory neuron cells obtained by serum induction method.

### (4.4) Electrophysiological activity assay of sensory neuron cells

(4.4. 1) Spontaneous discharge signals of induced sensory neuron cells were detected by multi-channel electrodes. A 48-well or 96-well MEA system multi-channel electrode plate (AXION Biosystem, US) was coated with 100ng/ml polylysine (Poly-L-lysine, Sigma-Aldrich, P4707)in an incubator (Panasonic, MCO-18AC) at 37°C, 5% CO₂ for 12 hours; and then the poly-lysine-coated MEA multi-channel electrode plate was taken out, with the polylysine being removed, and then washed three times with sterile water. After that, a PBS solution containing 3µg/ml gelatin (laminin 521) as a nerve cell coating matrix, was added to the MEA multi-channel electrode plate, and then the plated was coated in a cell incubator (Panasonic, MCO-18AC)at 37°C, 5% CO₂ for 3 hours. After the MEA multi-channel electrode plate was completely coated, the induced sensory neuron precursor cells were seeded at 5×10⁴ cells per well. The culture was performed by the sensory neuron cell medium of Example 3.3. The detection of spontaneous neuron electrical signals was performed after 14 days (i.e. the early stage of neuron maturation). The MEA multi-channel electrode plate for culturing induced sensory neuron cells was placed in a MEA chamber, and the cell culture conditions were adjusted in AxIS Navigator 2.0.2 software to 37°C, 5% carbon dioxide, and run for 10 minutes until the chamber environment was stable . Cell spontaneous discharge signals were recorded with AxIS Navigator 2.0.2 software (AXION Biosystem, US). The results were shown in Figure 5a, and the above results all showed that by using the induction method of the present invention, the three sources of somatic cells could generate electrophysiological activity earlier, with higher discharge frequency and higher spontaneous potential, as compared with those by using the serum induction method. Taking the induction of mesenchymal cells as an example, the chemical small molecule induction method used in the present invention and the serum induction method were compared, and the results showed that the combination of chemical small molecules LY2157299, CHIR98014 and SU5402 in the present invention could produce higher electrophysiological activity.

(4.4.2) The MEA plate and cells were prepared by the method of (4.4.1), and cultured for 20 days (i.e. the neuron mature period). 3µM tetrodotoxin (TOCRIS, 1078/1) was used as non-specific sodium ion inhibitor to verify the electrophysiological activity of sodium ion channels on cells. The MEA plate was placed in a MEA chamber, and the cell culture condition was adjusted to 37°C, 5% carbon dioxide, running for 10 minutes until the chamber environment was stable, and then the spontaneous discharge of cells was detected. The cell electrode plate was taken out, and added with 3µM tetrodotoxin per well for 10 minutes of reaction, and then the MEA plate was placed in the MEA chamber; the cell culture conditions were adjusted to 37°C, 5% carbon dioxide, running for 10 minutes until the chamber environment was stable; the spontaneous discharge of cells under the action of 3µM tetrodotoxin was detected. Cell spontaneous discharge signals were recorded with AxIS Navigator 2.0.2 software (AXION Biosystem, US). Data analysis was performed by AxIS Metrictool and NruralMethics software (AXION Biosystem, US). The results were shown in Figure 6. The non-specific sodium ion inhibitor had an electrical signal inhibitory effect on the sensory neuron cells (three somatic cell sources) induced by the method of the present invention, indicating that the sensory neurons induced by the method of the present invention could express sodium ion channel proteins, and the sodium channel proteins had an electrophysiological activity and could produce corresponding electrophysiological response to the sodium ion blocker. The results showed that, as compared with the serum control method, the sensory neurons produced by the method of the present invention had higher sodium ion channel activity under the same concentration of sodium ion blockers.

(4.4.3) The MEA plate and cells were prepared by the method of (4.4.2), and PF-05089771 (TOCRIS, 5931), a specific inhibitor of sodium ion channel 1.7, at a concentration of 1µM was used to verify the electrophysiological activity of sodium ion channels 1.8 and 1.9 on cells. The MEA plate was placed in a MEA chamber, and the cell culture conditions were adjusted to 37°C, 5% carbon dioxide, running for 10 minutes until the chamber environment was stable, and then the spontaneous discharge of cells was detected. The cell electrode plate was taken out, and added with 1µM PF-05089771 per well, and then the MEA plate was placed in the MEA chamber; the cell culture conditions were adjusted to 37°C, 5% carbon dioxide, running for 10 minutes until the chamber environment was stable; the spontaneous discharge of cells under the action of 1µM PF-05089771 was detected. Cell spontaneous discharge signals were recorded with AxIS Navigator 2.0.2 software (AXION Biosystem, US). Data analysis was performed by AxIS Metrictool and NruralMethics software (AXION Biosystem, US). Taking the induction of fibroblasts as an example, the chemical small molecule induction method used in the present invention and the serum induction method were compared, and the results were shown in Figure 7. The results showed that the concentration of LY2157299, CHIR98014 or SU5402 significantly improved the electrophysiological strength of sodium ion channels. Compared with the sensory neuron cells obtained by serum induction method, the sensory neuron cells obtained by the method of the present invention had a higher antagonistic effect on PF-05089771.

### Example 5. Effects of different types and concentrations of small molecules on the formation of induced sensory neuron cells

### (5.1) Morphology of peripheral sensory cells induced by different LY2157299 concentrations

The steps of Examples 3-4 were repeated by taking CD34+ -derived cells as an example, and the effects of different concentrations of LY2157299 on sensory neuron cells were observed, including the effect on the induction of sensory nerve precursor cells, and the effect on the expression of specific markers of sensory neuron cells, and the effect on the electrophysiological activity.

The results were shown in Figures 8a-8g. Figure 8g showed that the small-molecule inhibitor LY2157299 exhibited a linear increase in concentration versus protein expression in the expression of various sodium ion channel proteins, especially at the concentration of 7.5µM and 25µM, the expressions of SCN9A and SCN11A were significantly increased compared with the serum induction method. At LY2157299 concentrations of below 50 nM, the ion channel protein expressions were significantly lower than normal levels. As can be seen from the figures, the small molecule LY2157299 at different concentrations in the present invention can significantly promote the expressions of specific marker sodium ion channels 1.7 and 1.9 in peripheral neurons, and the neurons induced by using LY2157299 showed more antagonism against PF-05089771 (consistent with the results in Figure 7) (Note: The expression level of each group in the serum induction method was taken as 1, and the relative expression folds of the two groups of markers in the sensory nerve precursor cells induced by the induction medium with different component concentrations were calculated respectively. The same below). The image results above could represent induced sensory neuron cells derived from mesenchymal cells and fibroblasts.

(5.2) Taking CD34+-derived cells as an example, different small molecules (5µM RepSox, 5µM SB525334 and 5µM TEW-7197 molecules) were used to replace the LY2157299 in the sensory neuron induction medium N2GS212I-2 in Section (3.1) of Example 3; steps in Section (3.1) of Example 3 were repeated to observe the effects of different small molecules on the induced sensory nerve precursor cells. The results were shown in Figure 9.

(5.3) Taking CD34+-derived cells as an example, steps of Example 4 (4.3) were repeated by using the sensory nerve precursor cells obtained in (5.2), and the effects of different small molecules on the expression of neuronal precursor cell markers during the formation of sensory nerve precursor cells were observed. The results were shown in Figure 9f. As shown in the figures, the different inhibitor molecules were not only able to form neuron precursor cells (forming a neural plate structure shown in the figure), but also able to increase the expressions of neuron precursor cell markers.

### (5.4) Effects of different LDN concentrations on the formation of induced sensory neuron cells

The steps of Examples 3-4 were repeated by taking CD34+ -derived cells as an example, and the effects of different concentrations of LDN (that is, low concentration (12nM), medium concentration (50nM) and high concentration (180nM)) on sensory neuron cells were observed, including the effect on the induction of sensory nerve precursor cells, and the effect on the expression of specific markers of sensory neuron cells, and the effect on the electrophysiological activity. The results were shown in Figures 10a-10d. Capase3 was a marker of apoptosis. As shown in Figure 10, when the concentration of LDN193189 increased to 180nM or more, the expression of Caspase3 in the induced neuron cells was more than 1.5 times that of the serum control group; on the contrary, when the concentration of LDN193189 decreased to below 12nM, the expression of peripheral neuron-specific ion channel protein sodium ion channel 1.8 (SCN10A) was lower than that in serum control. In conclusion, 12nM-180nM was the optimal range. The images above could represent induced sensory neuron cells derived from mesenchymal cells and fibroblasts.

### (5.5) Effects of different N2 concentrations on the formation of induced sensory neuron cells

The steps of Examples 3-4 were repeated by taking CD34+ -derived cells as an example, and the effects of different concentrations of N2 (that is, low concentration (0.5%), medium concentration (0.5%) and high concentration (2%)) on sensory neuron cells were observed, including the effect on the induction of sensory nerve precursor cells, and the effect on the expression of specific markers of sensory neuron cells, and the effect on the electrophysiological activity. The results were shown in Figures 11a-11d. Capase3 was a marker of apoptosis. At 0.2% and 2.2% N2 concentration, the expression of Caspase3 in the induced neuron cells was more than 1.5 times that of the serum control group; on the contrary, the expression of peripheral neuron-specific ion channel protein sodium ion channel 1.8 (SCN10A) at 0.2% and 2.2% N2 concentration was lower than that of the serum control. In conclusion, the concentration range of 0.5%-2% was the optimal range; the above images could represent induced neuron cells derived from mesenchymal cells and fibroblasts.

### (5.6) Effects of different GS21 concentrations on the formation of induced sensory neuron cells

The steps of Examples 3-4 were repeated by taking CD34+ -derived cells as an example, and the effects of different concentrations of GS21 (that is, low concentration (0.2%), medium concentration (2.0%) and high concentration (3.1%)) on sensory neuron cells were observed, including the effect on the induction of sensory nerve precursor cells, and the effect on the expression of specific markers of sensory neuron cells, and the effect on the electrophysiological activity. The results were shown in Figures 12a-12d. Capase3 was a marker of apoptosis. At the 0.05% and 4% GS21 concentration, the expression of Caspase3 in the induced neuron cells was more than 1.5 times that of the serum control group; whereas the effect of GS21 on the peripheral neuron-specific ion channel protein sodium ion channel 1.8 (SCN10A) was not significantly different from that in serum control. In conclusion, the concentration range of 0.2%-3.1% was the optimal range; the above images could represent induced neuron cells derived from mesenchymal cells and fibroblasts.

### (5.7) Effects of different concentrations of glycine-glutamine monohydrate on the formation of induced sensory neuron cells

The steps of Examples 3-4 were repeated by taking CD34+ -derived cells as an example, and the effects of different concentrations of glycine-glutamine monohydrate (that is, low concentration (5mM), medium concentration (12mM) and high concentration (22mM)) on sensory neuron cells were observed, including the effect on the induction of sensory nerve precursor cells, and the effect on the expression of specific markers of sensory neuron cells, and the effect on the electrophysiological activity. The results were shown in Figures 13a-13c. The induced peripheral neurons prepared by using low (5 mM), medium (12 mM) and high (22 mM) concentrations of glycine-glutamine monohydrate had a normal morphology, but had a slow axonal growth at high concentration (22 mM).

In order to observe the effect of different concentrations of glycine-glutamine monohydrate on osmotic pressure, the effect of different concentrations of glycine-glutamine monohydrate on the osmotic pressure of culture system was detected by using an automatic freezing point osmometer (FM-8P, Shanghai Medical University Instrument Co., Ltd.), and specific operations were performed according to the product manual (FM-8P, Shanghai Medical University Instrument Co., Ltd.). The test results were shown in Figure 13d. Figure 13d showed that with the increase of glycine-glutamine monohydrate concentration, the osmotic pressure increased linearly. When the concentration exceeded 22 mM, the osmotic pressure of the culture system exceeded the threshold osmotic pressure 320 mOsm/kg for cell culture, indicating that the concentration of glycine-glutamine monohydrate used in the present invention was the most suitable concentration for cell culture.

### (5.8) Effects of different HEPES buffer concentrations on the formation of induced sensory neuron cells

The steps of Examples 3-4 were repeated by taking CD34+ -derived cells as an example, and the effects of different concentrations of HEPES buffer (that is, low concentration (5mM), medium concentration (10mM) and high concentration (15mM)) on sensory neuron cells were observed, including the effect on the induction of sensory nerve precursor cells, and the effect on the expression of specific markers of sensory neuron cells, and the effect on the electrophysiological activity. The results were shown in Figures 14a-14c.

Steps in (5.7) were repeated to observe the effect of the medium with different HEPES buffer concentrations on the osmotic pressure. The results were shown in Figure 14d. As shown, the cells were morphologically normal, and cell intolerance began to appear at high concentration (15 mM). With the increase of the concentration, the osmotic pressure of the culture system increased linearly. When the concentration was more than 15 mM, the osmotic pressure of the culture system exceeded the threshold osmotic pressure for cell culture, 320 mOsm/kg. Therefore, the HEPES buffer concentration determined in the present invention was the optimal concentration. The images above could represent sensory neuron cells derived from mesenchymal cells and fibroblasts.

### (5.9) Effects of different CHIR98014 concentrations on the formation of peripheral sensory neuron cells

The steps of Examples 3-4 were repeated by taking CD34+ -derived cells as an example, and the effects of different concentrations of CHIR98014 (0.3nM-5µM) on sensory neuron cells were observed, including the effect on the induction of sensory nerve precursor cells, and the effect on the expression of specific markers of sensory neuron cells.

The results were shown in Figures 15a-15g. Figures 15a-15f showed the sensory neuron cells obtained by different concentrations of CHIR98014. Figure 15g showed the effects of different concentrations of CHIR98014 on the expressions of peripheral sensory neuron-specific genes by using Q-PCR analysis. As compared with the serum induction method, the expression of SCN11A (Nav1.9) was significantly increased at the CHIR98014 concentration of 0.5µM to 3µM. However, when the CHIR98014 concentration was lower than 0.3nM and higher than 5µM, the expression of ion channel protein was lower than normal level; when the CHIR98014 concentration was higher than 5µM, the expression of neuron marker Tuj was lower than that at other concentrations. wherein, the expression data of SCN11A (N av 1. 9) were consistent with the data of strong antagonistic effect on PF-05089771 (Figure 7). The images above could represent sensory neuron cells derived from mesenchymal cells and fibroblasts.

### (5.10) Effects of different SU5402 concentrations on the formation of peripheral sensory neuron cells

The steps of Examples 3-4 were repeated by taking CD34+ -derived cells as an example, and the effects of different concentrations of SU5402 (that is, low concentration (3µM), medium concentration (6µM) and high concentration (10µM)) on sensory neuron cells were observed, including the effect on the induction of sensory nerve precursor cells, and the effect on the expression of specific markers of sensory neuron cells, and the effect on the electrophysiological activity. The results were shown in Figures 16a-16d.

Figures 16a-16c showed that cells could form axons at low concentration (3µM), medium concentration (6µM) and high concentration (10µM) of SU5402; Figure 16d showed the effects of different concentrations of SU5402 on the expressions of peripheral sensory neuron-specific genes by using Q-PCR analysis. As compared with that in the serum induction method, when the concentration of SU5402 was lower than 3µM, the expression of ion channel protein was lower than the control level; when the concentration was higher than 15µM, the cell death marker CASP3 was significantly increased. wherein, the expression data of SCN11A (Nav1.9) were consistent with the data of strong antagonistic effect on PF-05089771 (Figure 7). The images above could represent sensory neuron cells derived from mesenchymal cells and fibroblasts.

### (5.11) Effects of different DAPT concentrations on the formation of peripheral sensory neuron cells

The steps of Examples 3-4 were repeated by taking CD34+ -derived cells as an example, and the effects of different concentrations of DAPT (that is, low concentration (3µM), medium concentration (6µM) and high concentration (10µM)) on sensory neuron cells were observed, including the effect on the induction of sensory nerve precursor cells, and the effect on the expression of specific markers of sensory neuron cells, and the effect on the electrophysiological activity. The results were shown in Figures 17a-17f.

Figures 17a-17c showed that cells were able to form axons at low (3µM), medium (6µM) and high (10µM) concentrations of DAPT. Figure 17d showed the effects of different concentrations of DAPT on the expressions of peripheral sensory cell-specific genes by using Q-PCR analysis. When the DAPT concentration was lower than 3µM, the induced neuron cell markers and peripheral sensory neuron cell markers were much lower than those in the serum control group; on the contrary, when the DAPT concentration increased to more than 10µM, the expression of peripheral sensory neuron cell-specific ion channel protein sodium ion channel 1.8 (SCN10A) was decreased, while the expression of neuron marker Tuj was increased, indicating that high concentrations of DAPT promoted the generation of non-peripheral sensory neuron cells. The above data demonstrated that the DAPT concentration range used in the present invention was the most suitable for the production of peripheral sensory neuron cells. The images above could represent induced neuron cells derived from mesenchymal cells and fibroblasts.

In conclusion, as compared with the known methods, in the absence of serum components, the combination of small molecules and concentrations thereof in the present invention significantly promoted the expressions of various functional ion channels in peripheral neuron cells.

### Example 6. Quantitative detection of sensory neuron cell viability

The cell viability was quantitatively detected by Cyquant assay to compare the effects of different combinations of small molecule compounds on maintaining physiological states of cells during long-term culture of induced sensory neurons.

Cell plate coating was performed in a 96-well opaque cell culture plate according to the method in Example 4 (4.4.1). After the coating was completed, the induced nerve precursor cells were seeded at 5×10⁴ cells per well, and three parallel replicates were set up (the average of the three groups was calculated as the data). The cells were cultured in the sensory neuron cell induction medium of Example 3 with different concentrations of NT3, and the culture conditions were 37° C, 5% carbon dioxide, and the medium was half quantity changed every three days. The medium in the known method was used as a control group. Samples were taken at day 1, day 3, day 5 and day 10, respectively, and the cell viability detection was carried out by using CyQuant Kit (Invitrogen, X12223) according to the instruction manual, and date were read by SpectraMax i3 Multi-Mode Microplate Reader (VWR, ID3-STD). Taking the induced sensory neurons derived from fibroblasts as an example, the results were shown in Figure 18. The added NT3 in the present invention was able to effectively increase the in vitro viability of the induced neuron in the long-term culture process as compared with that of the sensory neuron cell CK obtained by the serum induction method.

## Claims

1. Use of LY2157299, RepSox, SB5253334 or TEW7179 in the induction of pluripotent stem cells to differentiate into sensory nerve precursor cells and sensory neuron cells.

2. The use of claim 1, wherein the dosage of LY2157299, RepSox, SB5253334 or TEW7179 is 55nM-25µM, preferably 100nM, 300nM, 500nM, 700nM, 900nM, 1µM, 2.5µM, 5µM, 7.5µM, 10µM, 12.5µM, 15µM, 17.5µM, 20µM or 22µM.

3. Use of human-NT3 or CHIR98014 in culturing sensory neurons.

4. The use of claim 3, wherein the dosage of human-NT3 is 0.1-49ng/ml, or the dosage of CHIR98014 is 0.5nM-3µM.

5. A sensory neuron cell induction medium, **characterized in that** a basal medium is formed by 50% (v/v) DMEM medium and 50% (v/v) neural basal medium, then the basal medium is supplemented with 0.5%-2% (v/v) N2 supplement (preferably 0.7%, 0.9%, 1.0%, 1.1%, 1.2%, 1.3%, 1.4%, 1.5%, 1.6%, 1.7% (v/v)), 0.2%-3.1% (v/v) GS21 supplement or B27 supplement (preferably 0.4%, 0.6%, 0.8%, 1.0%, 1.2%, 1.4%, 1.6%, 1.8%, 2.0%, 2.2%, 2.4%, 2.6%, 2.8%, 3.0% (v/v)), 0.1-15 mM HEPES buffer (preferably 1, 5, 10 mM), 5-22 mM glycine-glutamine monohydrate (preferably 10, 15, 20 mM), 12-180 nM LDN-193189 or LDN-193189 2HCl (preferably 20, 50, 70, 80, 90, 100, 110, 120, 150, 170nM), and 55nM-25µM LY2157299, RepSox, SB5253334 or TEW7179 (preferably 100nM, 300nM, 500nM, 700nM, 900nM , 1µM, 2.5µM, 5µM, 7.5µM, 10µM, 12.5µM, 15µM, 20µM or 22µM).

6. The sensory neuron cell induction medium of claim 5, **characterized in that** a basal medium is formed by 50% (v/v) DMEM medium and 50% (v/v) neural basal medium, then the basal medium is supplemented with 1% (v/v) N2 supplement, 2% (v/v) GS21 supplement or B27 supplement, 0.5mM HEPES buffer, 20mM glycine-glutamine monohydrate, 100nM LDN-193189 or LDN-193189 2HCl, and LY2157299, RepSox, SB5253334 or TEW7179 selected from 5µM, 7.5 µM or 12.5µM.

7. The sensory neuron cell induction medium of claim 5 or 6, wherein the induction medium is formed as follows: a basal medium is formed by 50% (v/v) DMEM medium and 50% (v/v) neural basal medium, then supplemented with 1% (v/v) N2 supplement, 2% (v/v) GS21 supplement or B27 supplement, 0.5mM HEPES buffer, 20mM glycine-glutamine monohydrate, 100nM LDN-193189 or LDN-193189 2HCl, and LY2157299 selected from 5µM, 7.5 µM or 12.5µM; preferably, the sensory neuron cell induction medium is further supplemented with serum replacement (such as serum albumin, transferrin, fatty acid), more preferably, the serum replacement is in purified form.

8. A sensory neuron cell medium, **characterized in that** the induction medium of any one of claims 5-7 is supplemented with 3-10µM SU5402 (preferably 5µM, 6µM, 7µM, 8µM, 9µM), 3-10µM DAPT (4µM, 5µM, 6µM, 7µM, 8µM, 9µM), 1-3µM CHIR99021 (preferably 1.3µM, 1.5µM, 1.7µM, 1.9µM, 2.0µM, 2.2µM, 2.5µM, 2.7µM) or 0.5nM-3µM CHIR98014 (preferably 5nM, 50nM, 100nM, 200nM, 300mM, 500mM, 700mM, 900mM, 1µM, 1.4µM, 1.8µM, 2.0µM, 2.5µM, 2.7µM), and 0.1-49ng/ml human-NT3 (preferably 1, 5, 10, 15, 20, 25, 30, 35, 40, 45 ng/ml), preferably, the sensory neuron cell medium is further supplemented with serum replacement (e.g., serum albumin, transferrin, fatty acid), more preferably, the serum replacement is in purified form.

9. A method for inducing pluripotent stem cells to differentiate into sensory neuron cells, **characterized in that** the pluripotent stem cells are cultured in the induction medium of any one of claims 5-7 to obtain sensory nerve precursor cells, and then the sensory nerve precursor cells are cultured in the sensory neuron cell medium of claim 8 to obtain sensory neuron cells, preferably, the culture is performed in the presence of a basal membrane, more preferably, the basal membrane is composed of one or more of Matrigel (STEMCELL Technologies), Laminin and Vitronectin.

10. Sensory nerve precursor cells, which are prepared by culturing pluripotent stem cells by using the induction medium of any one of claims 5-7.

11. Sensory neuron cells, which are prepared by culturing the sensory nerve precursor cells of claim 10 by using the sensory neuron cell medium of claim 8.

12. A method for inducing sensory neuron cells comprising, culturing pluripotent stem cells by the induction medium of any one of claims 5-7 to obtain sensory nerve precursor cells, and then culturing the sensory nerve precursor cells by the sensory neuron cell medium of claim 8.

13. The method of claim 9 or 12, wherein the pluripotent stem cells are prepared from somatic cells by using a reprogramming medium, preferably, wherein the reprogramming medium consists of a DMEM-F12 basal medium and supplementary components, and the supplementary components include 60 µg/mL-180 µg/mL L-ascorbic acid, 5.3 µmol/L-74 µmol/L hydrocortisone, 3 ng/mL-89 ng/mL sodium selenite, 8 µmol/L-23 µmol/L Optiferrin, 0.5 µmol/L-7.4 µmol/L retinyl acetate, 40 ng/mL-60 ng/mL plant-derived recombinant human basic growth factor, 8 µg /mL-12 µg/mL IGF, 0.2 µmol/L-0.6 µg/mL A-83, 2 µmol/L-6 µmol/L CHIR99021, and 100 µmol/L-450 µmol/L sodium butyrate.

14. The method of claim 13, **characterized in that** the supplementary components include 70 µg/mL-90 µg/mL L-ascorbic acid, 40 µmol/L-60 µmol/L hydrocortisone, 10ng/mL-30ng/mL sodium selenite, 8µmol/L-12µmol/L Optiferrin, 3µmol/L-6µmol/L retinyl acetate, 45 ng/mL-55 ng/mL plant-derived recombinant human basic growth factor, 8 µg/mL-12 µg/mL IGF, 0.3 µmol/L-0.5 µg/mL A-83, 3 µmol/L-5 µmol/L CHIR99021 and 280 µmol/L-410 µmol/L sodium butyrate.

15. The method of claim 13, **characterized in that** the supplementary components comprise 80 µg/mL L-ascorbic acid, 50 µmol/L hydrocortisone, 20 ng/mL sodium selenite, 10 µmol/L Optiferrin, 4 µmol/L retinyl acetate, 50 ng/mL plant-derived recombinant human basic growth factor, 10 µg/mL IGF, 0.4 µg/mL A-83, 4 µmol/L CHIR99021 and 400 µmol/L sodium butyrate.

16. The method of any one of claims 12-15, wherein the culturing is performed in the presence of a basal membrane, preferably, the basal membrane is composed of one or more of Matrigel (STEMCELL Technologies), Laminin and Vitronectin.

17. The method of any one of claims 13-16, wherein the somatic cells are human mesenchymal cells, human CD34+ cells or human fibroblasts (e.g. skin fibroblasts, lung fibroblasts, bladder fibroblasts, foreskin fibroblasts, uterine fibroblasts).

18. Use of the sensory neuron of claim 11 in screening a medicine for treating or alleviating pain or preparing a model for screening a medicine for treating or alleviating pain.
